# EUROPEAN PATENT APPLICATION

(11) **EP 3 012 323 A1**
(43) Date of publication of application: **27.04.2016**
(21) Application number: 14813420.8
(22) Date of filing: 18.06.2014
(51) Int. Cl.: C12N 15/09, A01H 1/00, A01H 1/06, A01H 5/00, C12Q 1/68

(54) **GENE ENCODING ENZYME CAPABLE OF OXIDIZING POSITION-16 IN STEROID BACKBONE, AND PLANT BODY IN WHICH EXPRESSION OF SAID GENE IS REDUCED**

(30) Priority: 18.06.2013 JP 2013127901
(71) Applicant: KIRIN COMPANY, LIMITED, Tokyo 164-001 (JP)
(72) Inventor: UMEMOTO Naoyuki, Tokyo 164-0001 (JP)
(74) Representative: Hoffmann Eitle
(86) International application number: PCT/JP2014/066094
(87) International publication number: WO 2014/203914

(57) **Abstract**

This invention provides a plant belonging to the family *Solanaceae* that does not produce glycoalkaloids. This invention concerns a protein having activity of an enzyme that oxidizes position 16 of the steroid skeleton of a plant belonging to the family *Solanaceae,* a novel plant in which a gene encoding such protein is suppressed, and a method for producing and testing such plant.

SEQUENCE LISTING

<110>  KIRIN COMPANY, LIMITED

<120>  A gene which encodes an enzyme which reduces steroid backbone at
       position 16 and a plant in which the expression of said gene is
       reduced

<130>  PH-5881-PCT

<150>  JP 2013-127901
<151>  2013-06-18

<160>  13

<170>  PatentIn version 3.5

<210>  1
<211>  341
<212>  PRT
<213>  Solanum tuberosum

<400>  1

Met Ala Glu Leu Leu Ser Asn Trp Ser Ser Thr Leu Glu Ala Val Pro
1               5                   10                  15

Pro Ser His Cys Ile Pro Val His Glu Arg Pro Ser Asp Thr Val Glu
            20                  25                  30

Ile Val Asp Thr Ile Pro Val Ile Asp Leu Gly Lys Ala Asn Gly Glu
        35                  40                  45

Glu Arg Ser Ala Val Val Lys Asp Leu Leu Lys Ala Phe Glu Glu Tyr
    50                  55                  60

Gly Phe Phe Gln Ile Ile Asn His Gly Val Pro Val Asp Leu Met Asp
65                  70                  75                  80

Glu Ala Met Lys Val Tyr Lys Glu Phe Phe Ser Leu Pro Ala Glu Glu
                85                  90                  95

Lys Ala Glu Tyr Ala Lys Asp Ala Ala Asn Asp Thr Asn Arg Gly Ala
            100                 105                 110

Ala Thr Leu Tyr Ser Ser Ser Ala Lys His Tyr Asp Ser Glu Glu His
        115                 120                 125

Arg Tyr Trp Arg Asp Val Leu Glu His Ser Cys Asn Leu Asp Gly Lys
    130                 135                 140

Asp Lys Lys Thr Trp Pro Ser Asn Pro Pro Arg Tyr Arg Glu Val Ile
145                 150                 155                 160

Gly Ala Tyr Gly Asp Glu Leu Arg Arg Val Ser Lys Val Ile Leu Gly
                165                 170                 175

Leu Leu Ala Glu Gly Leu Gly Leu Glu Ala Gly Phe Phe Asp Thr Glu
            180                 185                 190

Leu Gly Gln Arg Met Leu Val Asn His Tyr Pro Ala Cys Pro Asp Pro
        195                 200                 205

Ser Leu Thr Leu Gly Val Gly Gly His Cys Asp Pro Asn Leu Ile Thr
    210                 215                 220

Ile Ile Gln Gln Glu Val Tyr Gly Leu Gln Ile Leu Lys Asp Asp Lys
225                 230                 235                 240

Trp Ile Gly Val Gln Pro Ile Arg Asn Ala Phe Val Val Asn Ser Gly
                245                 250                 255

Leu Pro Ile Thr Val Val Ser Asn Gly Lys Leu Thr Ser Val Ala His
            260                 265                 270

Arg Val Val Thr Asn Thr Thr His Ser Arg Thr Ser Ile Gly Thr Phe
        275                 280                 285

Ile Cys Pro His Asp Ile Val Glu Pro Ala Lys Ala Leu Val Gly Pro
    290                 295                 300

Glu Asn Pro Pro Gln Phe Lys Ser Phe Asn Trp Gly Ile Asp Phe Met
305                 310                 315                 320

Pro His Tyr Leu Ser Lys Lys Ser Val Tyr His Ala Ser Leu Glu Pro
                325                 330                 335

Phe Lys Ile Asp Ala
            340

<210>  2
<211>  1023
<212>  DNA
<213>  Solanum tuberosum

<400>  2
atggcggagc ttctttcaaa ctggtcaagc acattagaag cagttcctcc aagtcattgc      60

atcccagtgc atgaaagacc atcggataca gttgaaattg tggacactat tccagtcatt     120

gatttgggaa aagctaatgg tgaagaacga agtgctgttg ttaaagatct tttgaaagct     180

tttgaagaat atgggttttt tcagataatc aatcatggag tacccgtaga tctaatggat     240

gaagcaatga aagtgtacaa agaatttttc agtctgccag cagaagagaa agcagaatat 300

gcaaaggatg cagctaatga tacaaatagg ggtgcagcta cactgtacag tagcagcgct 360

aagcattatg attcagagga gcatcgttac tggagagatg tcttggaaca tagctgcaat 420

cttgatggga aagacagaaa aacttggcct agtaaccctc caagatatag ggaggttatt 480

ggtgcatatg gagatgaatt gagaagggtg agcaaagtta tcttgggtct gttagctgaa 540

gggctaggtt tggaggcagg gttctttgac acagaacttg ggcagagaat gcttgtgaat 600

cactatccag catgcccaga tccaagttta accttgggag ttggtggaca ttgtgatcct 660

aatctcataa ccattatcca acaagaagtg tatggtcttc aaatattgaa ggatgacaaa 720

tggattggtg tgcagcctat ccaaaatgca tttgtggtca attctggttt accaattacg 780

gtagttagca atggaaagct aactagtgtt gcacatcgtg tggtgacaaa cacaactcat 840

tcacgaacct ccattggtac ttttatttgc ccacacgata ttgttgaacc tgcaaaagca 900

cttgttggtc cggagaatcc tccacagttc aaatccttta attggggaat tgattttatg 960

ccacattacc tcagcaagaa atcagtttac cacgcatcat tggagccctt caaaatcgat 1020

gct 1023

<210> 3
<211> 342
<212> PRT
<213> Solanum lycopersicum

<400> 3

Met Ala Asp Leu Leu Ser Asn Trp Ser Ser Thr Leu Glu Ala Val Pro
1               5                   10                  15

Lys Ser His Cys Ile Pro Glu His Glu Arg Pro Ser Asp Pro Val Glu
            20                  25                  30

Ile Gly Asp Ser Ile Pro Val Ile Asp Leu Gly Lys Ala Asn Gly Glu
        35                  40                  45

Glu Arg Ser Val Val Val Lys Asp Leu Leu Lys Ala Phe Glu Glu Tyr
    50                  55                  60

Gly Phe Phe Gln Ile Ile Asn His Gly Val Pro Val Asp Leu Met Asp
65                  70                  75                  80

Glu Ala Met Lys Val Tyr Lys Glu Phe Phe Ser Leu Pro Ala Glu Glu
                85                  90                  95

Lys Glu Asn Tyr Ala Lys Asp Ala Ala Asn Asn Thr Asn Arg Gly Ala
            100                 105                 110

Ala Thr Leu Tyr Ser Ser Ser Ala Lys His Tyr Asp Ser Glu Glu His
        115                 120                 125

Arg Tyr Trp Arg Asp Val Leu Glu His Ser Cys Asn Leu Asp Gly Glu
    130                 135                 140

Asp Lys Lys Thr Trp Pro Asp Asn Pro Pro Arg Tyr Arg Glu Val Ile
145                 150                 155                 160

Gly Ala Tyr Gly Asp Glu Leu Arg Arg Val Ser Lys Val Ile Leu Gly
                165                 170                 175

Met Leu Ser Glu Gly Leu Gly Leu Glu Ala Gly Phe Phe Asp Lys Glu
            180                 185                 190

Leu Gly Gln Arg Met Leu Val Asn His Tyr Pro Ala Cys Pro Asn Pro
        195                 200                 205

Ser Leu Thr Leu Gly Val Gly Gly His Cys Asp Pro Asn Leu Ile Thr
    210                 215                 220

Ile Ile Gln Gln Glu Val Tyr Gly Leu Gln Ile Leu Lys Asp Asp Lys
225                 230                 235                 240

Trp Ile Gly Val Gln Pro Ile Arg Asn Ala Phe Val Val Asn Ser Gly
                245                 250                 255

Leu Pro Ile Thr Val Tyr Ser Asn Gly Lys Leu Thr Ser Val Ala His
            260                 265                 270

Arg Val Val Thr Asn Thr Thr Glu Ser Arg Thr Ser Ile Gly Thr Phe
        275                 280                 285

Ile Cys Pro His Glu Ile Val Glu Pro Ala Lys Ala Leu Val Gly Pro
    290                 295                 300

Glu Asn Pro Pro Gln Phe Lys Pro Phe His Trp Gly Ile Asp Phe Met
305                 310                 315                 320

Pro His Tyr Leu Ser Lys Lys Ser Val Tyr His Ala Ser Leu Glu Pro
                325                 330                 335

Phe Lys Thr Glu Ala Asn
            340

<210>  4
<211>  1026

<212>  DNA
<213>  Solanum lycopersicum

<400>  4
atggcggatc ttctctcgaa ctggtcaagc acattagaag cagttcctaa aagtcattgc      60

atcccagagc atgaaagacc atcagatcca gttgaaattg gcgacagtat tccagtcatt     120

gatttgggaa aagctaatgg tgaagaacga agtgttgttg ttaaagatct gttgaaagct     180

tttgaagaat atgggttttt tcagataatc aatcatggag tacctgtaga tctaatggat     240

gaagcaatga aagtgtacaa agaatttttc agtcttccag ctgaagaaaa agaaaattat     300

gcaaaagatg cagctaataa taccaatagg ggtgcagcta cactgtacag tagcagtgct     360

aagcattatg attcagagga gcatcgttac tggagagatg tgttggaaca tagctgcaat     420

cttgatggag aagacaaaaa aacttggccc gataaccctc caagatatag ggaggttatt     480

ggtgcctatg gtgatgaatt gagaagggtg agcaaagtta tcttgggtat gttaagtgaa     540

gggctaggtt tggaggcagg gttctttgac aaagaacttg ggcagagaat gcttgtgaat     600

cactatccag catgtccaaa tccaagttta actttgggag ttggtggaca ttgtgatcct     660

aatctcataa ccattatcca acaagaagtc tatggtcttc aaatattgaa ggatgacaaa     720

tggattggtg tgcagcctat tcgcaatgca tttgtggtta attctggttt accaattacg     780

gtatatagca atggaaagct aactagtgtt gcacatcgtg tggtgacaaa cacaactgag     840

tcacgaacct ccattggtac ttttatttgc ccacatgaga ttgttgaacc tgcaaaagca     900

cttgttggtc ctgagaatcc tccacagttc aaacccttcc attggggaat cgattttatg     960

ccacattacc tcagcaagaa atcagtgtac cacgcttcat tggagccctt caaaacagaa    1020

gctaat                                                               1026


<210>  5
<211>  1271
<212>  DNA
<213>  Solanum tuberosum

<400>  5
atggcggacc ttctttcaaa ctggtcaagc acattagaag cagttcctcc aagtcattgc      60

atcccagtgc atgaaagacc atcggatcca gttgaaattg tggacaatat tccagtcatt     120

gatttgggaa aagctaatgg tgaagaacga agtgttgttg ttaaagaact tttgaaagct     180

tttgaagaat atgggttttt tcaggtttat tatttataca atagtacaac tctgttcttt     240

tttctttttt tttcttattg tatttaaaaa tgatctgaaa ttgaaatgat gaaatagata     300

atcaatcatg gagtacccgt agatctaatg gatgaagcaa tgaaagtgta caaagaattt     360

ttcagtctgc cagcagcaga gaaagcagaa tatgcaaagg atgcagctaa tgatacaaat     420

aggggtgcag ctacactgta cagtagcagc gctaagcatt atgattcaga ggagcatcgt     480

tactggagag atgtcttgga acatagctgc aatcttgatg ggaaagacaa aaaaacttgg 540

cctagtaacc ctccaagata taggtaccta cctaaactat gcttagcaaa attccctctt 600

gttatttttc ttacctagta tttgcttgtc cttcagggag gttattggtg catatggaga 660

tgaattgaga agggtgagca aagttatctt gggtctgtta gctgaagggc taggtttgga 720

ggcagggttc tttgacacag aacttgggca gagaatgctt gtgaatcact atccagcatg 780

cccagatcca agtttaacct tgggagttgg tggacattgt gatcctaatc tcataaccat 840

tatccaacaa gaagtgtatg gtcttcaaat attgaaggat gacaaatgga ttggtgtgca 900

gcctatccgc aatgcatttg tggtcaattc tggtttacca attacggtag gtgtaacact 960

ttctcttaat tttcatggtc tacaagcgat tctcttattg ctctgttttt tttgtataaa 1020

tacaggtagt tagcaatgga aagctaacta gtgttgcaca tcgtgtggtg acaaacacaa 1080

ctcattcacg aacctccatt ggtactttta tttgcccaca cgatattgtt gagcctgcaa 1140

aagcacttgt tggtccggag aatcctccac agttcaaatc ctttaattgg ggaattgatt 1200

ttatgccaca ttacctcagc aagaaatcag tttaccacgc atcattggag cccttcaaaa 1260

tcgatgctta a 1271

<210> 6
<211> 25
<212> DNA
<213> Artificial

<220>
<223> Primer

<400> 6
caccatggcg gagcttcttt caaac 25

<210> 7
<211> 22
<212> DNA
<213> Artificial

<220>
<223> Primer

<400> 7
ttaagcatcg attttgaagg gc 22

<210> 8
<211> 29
<212> DNA
<213> Artificial

<220>
<223> Primer

<400> 8
gagctctaga tttgggaaaa gctaatggt 29

<210>  9
<211>  25
<212>  DNA
<213>  Artificial

<220>
<223>  Primer

<400>  9
ggatccatat gcaccaataa cctcc                                          25


<210>  10
<211>  19
<212>  DNA
<213>  Artificial

<220>
<223>  Primer

<400>  10
taaagcacga ggaagcggt                                                 19


<210>  11
<211>  19
<212>  DNA
<213>  Artificial

<220>
<223>  Primer

<400>  11
gcacaacaga caatcggct                                                 19


<210>  12
<211>  26
<212>  DNA
<213>  Artificial

<220>
<223>  Primer

<400>  12
tggtttacca attacggtag ttagca                                         26


<210>  13
<211>  22
<212>  DNA
<213>  Artificial

<220>
<223>  Primer

<400>  13
ttaagcatcg attttgaagg gc                                             22

## Description

### Technical Field

The present invention relates to an enzyme that oxidizes position 16 of the steroid skeleton of a plant belonging to the family *Solanaceae,* such as a potato, DNA encoding the enzyme that oxidizes position 16 of the steroid skeleton, a method for breeding and selecting a novel plant belonging to the family *Solanaceae,* such as a potato, using such DNA, and a plant belonging to the family *Solanaceae,* such as a potato, which does not accumulate glycoalkaloids because of the suppressed expression of the gene encoding the enzyme or the altered activity of the enzyme.

### Background Art

Glycoalkaloids are a group of plant-derived compounds, which are also referred to as steroidal alkaloids. The glycoalkaloid structure is composed of C27 isoprenoids containing a nitrogen atom, and it has been reported that there are 422 compounds of glycoalkaloids from plants belonging to the genus *Solanum* (Non-Patent Literature 1, chapter 7.8). As to a plant other than those belonging to the genus *Solanum* in the family *Solanaceae,* some plants belonging to the family *Liliaceae* are also known to contain glycoalkaloids. Among glycoalkaloids of such plants, important ones are chaconine and solanine from potatoes *(Solanum tuberosum),* and tomatine from tomatoes (*Solanum lycopersicum*), which belong to the genus *Solanum* in the family *Solanaceae.*

Potato is the fourth most produced crop in the world following corn, rice, and wheat. However, it is a well-known fact that toxic chaconine and solanine are contained in the buds coming out of the tubers or the aerial parts of the plants. Symptoms of poisoning such as abdominal pain, dizziness, and mild disturbance of consciousness are caused by chaconine or solanine. Chaconine and solanine are easily accumulated in tubers when the tubers are damaged or exposed to solar light, and thus there is a risk of poisoning accident caused by improper management of tubers.

These poisoning accidents frequently happen, and recently, a glycoalkaloid poisoning accident occurred at an elementary school in Nara City, Japan on July 16, 2009 (reported by Asahi.com). Potatoes are usually safe foods because they are managed such that the content of glycoalkaloid is maintained at 20 mg/100 g or less by storing potato tubers in a dark place etc. However, in consideration of the risk of such a poisoning accident described above, reducing glycoalkaloids in potato is a matter of concern to all of the persons who deal with potatoes such as the breeding, production, storage, transportation, sale, and purchase of potatoes, but has not been achieved to date. The reasons are as follows. A wild potato species with no glycoalkaloids has not been found, the biosynthetic pathway of glycoalkaloids has remained unconfirmed (Figures 7.24A and 7.24B of Non-patent Literature 1, and Non-patent Literature 2), and the identification of genes involved in the biosynthetic pathway has not been proceeded.

Glycoalkaloids exhibit toxicity such as cholinesterase inhibitory activity or membrane disruption effect, but in addition to this, it is known that glycoalkaloids exhibit medicinal effects such as anti-cancer activity, a liver protective effect, an antispasmodic effect, an immune system promoting effect, an antibacterial effect, an antiprotozoal effect, and shellfish killing activity (Non-patent Literature 1). It has also been reported that esculeoside A, which is a metabolite of glycoalkaloids in tomato, exhibits various physiological effects (Non-patent Literature 3). However, research and development on suppressing the metabolites or efficient production thereof have hardly proceeded since the biosynthetic pathway thereof is not known.

Several enzyme genes catalyzing the transglycosylation process following the aglycone biosynthesis process have been reported (Non-patent Literature 4 to Non-patent Literature 6). However, in Non-patent Literature 4, the gene of UDP-galactosyltransferase, which mediates the conversion of solanidine, which is aglycone, to γ solanine, and a strain in which the gene is suppressed have been reported, but the production of chaconine has not been suppressed at all (Figure 2 of Non-patent Literature 4). In Non-patent Literature 4, the gene of UDP-glucosyltransferase, which mediates the conversion of solanidine to γ chaconine, and a strain in which the gene is suppressed have been reported, but the production of both chaconine and solanine is hardly suppressed (Figure 5 of Non-patent Literature 5). In Non-patent Literature 6, the gene of rhamnosyl transferase, which mediates the conversion of β chaconine to α chaconine and β solanine to α solanine, has been reported, but the β-form and γ-form are increased by the suppression of the gene, although the α-form is decreased. As seen from these, by the suppression of the transglycosylation process, the molecular species of glycoalkaloids can be changed but it is very difficult to control the total amount of glycoalkaloids.

There is a report of an attempt to decrease glycoalkaloids by overexpressing biosynthetic genes of plant sterols or plant hormones (Non-patent Literature 7). However, the amount of glycoalkaloids can only be reduced to about a half at most, and thus an effective means has not been provided in modifying the pathway (Figure 5 of Non-patent Literature 7).

Cholesterol had been considered to be a starting material of a glycoalkaloid biosynthesis pathway, and a process of oxidation at 3 positions (i.e., positions 16, 22, and 26) of the steroid skeleton had been predicted (Non-patent Literatures 8 and 9). In recent years, genes of 3 enzymes that catalyze the process of oxidation in the glycoalkaloid biosynthesis pathway (i.e., a C gene, a D gene, and an E gene) were reported (Patent Literatures 1 and 2). The C gene is deduced to be an oxidase at position 26, the D gene is deduced to be an oxidase at position 22, and the E gene is not deduced to be an enzyme that mediates the first oxidation at position 16 (Non-patent Literature 10 and Patent Literature 3). In addition, genes involved in glycoalkaloid biosynthesis, such as a Y gene as an aminotransferase gene (Patent Literature 4) and a DWF1H gene as a gene of an enzyme that reduces position 24 of the steroid skeleton (Patent Literature 5) have been reported.

### Prior Art Literature

### Patent Literatures

Patent Literature 1: WO 2011/025011
Patent Literature 2: WO 2012/029804
Patent Literature 3: WO 2013/077440
Patent Literature 4: WO 2013/073699
Patent Literature 5: WO 2012/157677

### Non-Patent Literatures

Non-Patent Literature 1: Eich, Solanaceae and Convolvulaceae: Secondary Metabolite, 2008, Springer
Non-Patent Literature 2: Ginzberg et al., Potato Research, 2009, 52: 1-15
Non-Patent Literature 3: Nohara et al., J. Nat. Prod., 2010, 73: 1734-1741
Non-Patent Literature 4: McCue et al., Plant Sci., 2005, 168: 267-273
Non-Patent Literature 5: McCue et al., Phytochemistry, 2006, 67: 1590-1597
Non-Patent Literature 6: McCue et al., Phytochemistry, 2007, 68: 327-334
Non-Patent Literature 7: Arnqvist et al., Plant Physiol., 2003, 131: 1792-1799
Non-Patent Literature 8: Eckart Eich, "Solanaceae and Convolvulaceae: Secondary Metabolite," 2008, Springer, Heidelberg, Germany, pp. 441-446
Non-Patent Literature 9: Kaneko et al., Phytochemistry, 1977, 16: 791-793
Non-Patent Literature 10: Umemoto et al., Abstract for Symposium of the 30th Annual Meeting of the Japanese Society of Plant Cell and Molecular Biology, Ikoma, 2012, p. 64

### Summary of the Invention

### Objects to Be Attained by the Invention

The present invention provides an enzyme that oxidizes position 16 of the steroid skeleton, DNA encoding the enzyme that oxidizes position 16 of the steroid skeleton, a method for breeding and selecting a novel plant belonging to the family *Solanaceae* using the DNA, and a plant belonging to the family *Solanaceae*, which does not produce cholesterols, including glycoalkaloids, because of the suppressed expression of the gene encoding the enzyme or the altered activity of the enzyme.

### Means for Attaining the Objects

The present inventor has conducted concentrated studies in order to attain the above objects. The present inventor focused on the possibility such that an enzymatic gene involved in oxidation of position 16 at the time of glycoalkaloid biosynthesis may not be cytochrome P-450 monooxygenase and discovered candidate genes *in silico.* He suppressed the expression of the endogenous candidate gene by causing expression of parts of the candidate genes to induce RNAi. As a result, he successfully obtained a potato transformant with significantly reduced glycoalkaloid content and, at the same time, identified the glycoalkaloid biosynthetic enzyme gene. Also, he demonstrated acquisition of a glycoalkaloid-free plant belonging to the family *Solanaceae* such as a potato by selecting a plant in which the expression of the above gene is suppressed. He also demonstrated that comparison of the genome sequence of the above-mentioned gene among various plants belonging to the family *Solanaceae* would enable the polymorphism analysis and production of a novel plant belonging to the family *Solanaceae.* This has led to the completion of the present invention. He also succeeded in producing tomatoes with reduced glycoalkaloid content through suppression of the endogenous gene in tomatoes in a similar manner.

Specifically, the present invention includes the following.
[1] A method for producing a plant with a reduced risk for accumulation of glycoalkaloids, comprising suppressing the activity of an enzyme that oxidizes position 16 of the steroid skeleton of a plant belonging to the family *Solanaceae* or suppressing the expression of a gene encoding the enzyme.
[2] The method according to [1], wherein the plant is a cultivated plant.
[3] The method according to [1] or [2], wherein the enzyme that oxidizes position 16 of the steroid skeleton of a plant belonging to the family *Solanaceae* is either protein (a) or (b) below:
   (a) a protein consisting of the amino acid sequence as shown in SEQ ID NO: 1 or 3; or
   (b) a protein consisting of an amino acid sequence derived from the amino acid sequence as shown in SEQ ID NO: 1 or 3 by deletion, substitution, insertion, or addition of one or several amino acids and oxidizing position 16 of the steroid skeleton of a plant belonging to the family *Solanaceae.*
[4] The method according to [1] or [2], wherein the enzyme that oxidizes position 16 of the steroid skeleton of a plant belonging to the family *Solanaceae* is encoded by a gene consisting of any of DNAs (c) to (f) below:
   (c) DNA consisting of the nucleotide sequence as shown in SEQ ID NO: 2 or 4;
   (d) DNA hybridizing under stringent conditions to DNA consisting of a nucleotide sequence complementary to DNA consisting of the nucleotide sequence as shown in SEQ ID NO: 2 or 4 and encoding a protein having activity of oxidizing position 16 of the steroid skeleton of a plant belonging to the family *Solanaceae;*
   (e) DNA consisting of a nucleotide sequence having 80% or higher sequence identity with the nucleotide sequence as shown in SEQ ID NO: 2 or 4 and encoding a protein that oxidizes position 16 of the steroid skeleton of a plant belonging to the family *Solanaceae*; and
   (f) DNA consisting of a degenerate isomer comprising the nucleotide sequence as shown in SEQ ID NO: 2 or 4.
[5] The method according to [1] or [2], wherein the enzyme that oxidizes position 16 of the steroid skeleton of a plant belonging to the family *Solanaceae* is protein (g) or (h) below:
   (g) a protein consisting of the amino acid sequence as shown in SEQ ID NO: 3; or
   (h) a protein consisting of an amino acid sequence derived from the amino acid sequence as shown in SEQ ID NO: 3 by deletion, substitution, insertion, or addition of one or several amino acids and oxidizing position 16 of the steroid skeleton of a plant belonging to the family *Solanaceae.*
[6] The method according to [1] or [2], wherein the enzyme that oxidizes position 16 of the steroid skeleton of a plant belonging to the family *Solanaceae* is encoded by a gene comprising any of DNAs (i) to (1) below:
   (i) DNA consisting of the nucleotide sequence as shown in SEQ ID NO: 4;
   (j) DNA hybridizing under stringent conditions to DNA consisting of a nucleotide sequence complementary to DNA consisting of the nucleotide sequence as shown in SEQ ID NO: 4 and encoding a protein that oxidizes position 16 of the steroid skeleton of a plant belonging to the family *Solanaceae;*
   (k) DNA consisting of a nucleotide sequence having 80% or higher homology to the nucleotide sequence as shown in SEQ ID NO: 4 and encoding a protein that oxidizes position 16 of the steroid skeleton of a plant belonging to the family *Solanaceae;* and
   (1) DNA consisting of a degenerate isomer of the nucleotide sequence as shown in SEQ ID NO: 4.
[7] The method according to any of [1] to [6], wherein the activity of the enzyme that oxidizes position 16 of the steroid skeleton of a plant belonging to the family *Solanaceae* or expression of a gene encoding such enzyme is suppressed by genetic recombination.
[8] The method according to any of [1] to [6], wherein the activity of the enzyme that oxidizes position 16 of the steroid skeleton of a plant belonging to the family *Solanaceae* or expression of a gene encoding such enzyme is suppressed by deletion of a gene that encodes the enzyme.
[9] The method according to any of [1] to [6], which comprises selecting a progeny obtained by crossing involving the use of a plant in which the activity of the enzyme that oxidizes position 16 of the steroid skeleton of a plant belonging to the family *Solanaceae* or expression of a gene encoding such enzyme is suppressed as a mother plant.
[10] The method according to [9], wherein the mother plant is obtained by artificial modification of the gene encoding an enzyme that oxidizes position 16 of the steroid skeleton of a plant belonging to the family *Solanaceae* via mutation.
[11] The method according to [9], wherein the mother plant is obtained by screening of wild-type plants.
[12] The method according to [9], wherein the mother plant is obtained and the progeny is selected by detecting the presence of a mutation or polymorphism in the gene encoding an enzyme that oxidizes position 16 of the steroid skeleton of a plant belonging to the family *Solanaceae.*
[13] The method according to [12], wherein the detection of the presence of a mutation or polymorphism comprises steps of:
   (i) isolating a nucleic acid, which is genomic DNA or RNA, from a plant;
   (ii) synthesizing cDNA by reverse transcription when the nucleic acid of (i) is RNA;
   (iii) amplifying a gene fragment comprising at least a part of the nucleotide sequence as shown in SEQ ID NO: 2, 4, or 5 from DNA obtained in the step (i) or (ii); and
   (iv) determining the presence of a mutation or polymorphism in DNA.
[14] A cultivated plant produced by the method according to any of [1] to [13].
[15] The cultivated plant according to [14], which is a potato.
[16] A gene marker sequence used in the method according to [13].
[17] A primer sequence for gene amplification used in the method according to [13].
[18] A cultivated plant comprising a mutation in a gene encoding an enzyme that oxidizes position 16 of the steroid skeleton of a plant belonging to the family *Solanaceae* and having a reduced risk of accumulation of glycoalkaloids.
[19] The cultivated plant according to [18], which does not accumulate glycoalkaloids.

The present specification encompasses the contents of the specification and/or drawings of JP patent Application No. 2013-127901, based on which the present application claims priority.

### Advantageous Effects of Invention

According to the present invention, the expression of the activity of a protein having activity of oxidizing position 16 of the steroid skeleton of a plant belonging to the family *Solanaceae* and that of the gene encoding such protein can be regulated. Specifically, the present invention provides a method for producing a plant in which the activity of such gene is regulated and a plant belonging to the family *Solanaceae* that does not accumulate glycoalkaloids.

### Brief Description of Drawings

Fig. 1 shows the results of analysis of sequence homology of the biosynthetic gene 16DOX between a potato and a tomato using GENETYX (Genetyx Corporation).
Fig. 2 shows the construction of a vector for 16DOX gene suppression, showing the internal constructions of the right border (RB) and left border (LB) and the restriction enzyme sites of T-DNA, which is the gene to be introduced.
Fig. 3 shows the glycoalkaloid content in the *in vitro* stems of potato transformants. Each error bar indicates the standard deviation.
Fig. 4 shows the results of RT-PCR of RNA extracted from the *in vitro* stems of potato transformants.
Fig. 5 shows the glycoalkaloid content in the tuber epidermis of potato transformants. Each error bar indicates the standard deviation.
Fig. 6 shows the glycoalkaloid content in young leaves of tomato transformants.

### Embodiments for Carrying out the Invention

Hereafter, the present invention is described in detail.

### 1. Enzymatic protein that oxidizes position 16 of steroid skeleton

The enzymatic protein that oxidizes position 16 of the steroid skeleton according to the present invention is a glycoalkaloid biosynthetic enzyme that is contained in a plant belonging to the family *Solanaceae,* such as a potato. Examples of plants of the family *Solanaceae,* such as *Solanum tuberosum,* include potatoes (*Solanum tuberosum*), tomatoes (*Solanum lycopersicum*), eggplants (*Solanum melongena*), and capsicum (*Capsicum annuum*). An example of a glycoalkaloid obtained with the aid of the enzyme of the present invention is a glycoalkaloid synthesized by a plant belonging to the family *Solanaceae,* such as a potato, and specific examples thereof include glycoalkaloids, such as chaconine and solanine of a potato, and glycoalkaloids, such as tomatine, of a tomato. The glycoalkaloid biosynthetic enzyme according to the present invention is a protein having an activity of an enzyme that oxidizes position 16 of the steroid skeleton, which is essential for glycoalkaloid synthesis. An example of a preferable steroid compound serving as a substrate for the glycoalkaloid biosynthetic enzyme according to the present invention is a cholesterol. Examples of cholesterols include cholesterol, sitosterol, campesterol, stigmasterol, brassicasterol, 22-hydroxycholesterol, 26-hydroxycholesterol, and 22-26-dihydroxycholesterol. The enzyme that oxidizes position 16 of the steroid skeleton according to the present invention oxidizes such substances. In the present description, such enzymatic activity is referred to as "activity of position-16-oxidizing enzyme," and an enzyme having such enzymatic activity is referred to as a "position-16-oxidizing enzyme." The enzymatic protein according to the present invention is a 2-oxoglutaric-acid-dependent dioxygenase.

Examples of position-16-oxidizing enzymes that can be used in the present invention include, but are not limited to, enzymes comprising the amino acid sequences as shown in SEQ ID NO: 1 and SEQ ID NO: 3, respectively, derived from potatoes and tomatoes. Further, a protein comprising an amino acid sequence having some mutations in the amino acid sequence as shown in SEQ ID NO: 1 or SEQ ID NO: 3 and having activity of position-16-oxidizing enzyme is within the scope of the enzyme that can be used in the present invention. A potato-derived position-16-oxidizing enzyme is referred to as "St16DOX," and a tomato-derived position-16-oxidizing enzyme is referred to as "S116DOX."

Examples of "an amino acid sequence having some mutations" include an amino acid sequence derived from the amino acid sequence as shown in SEQ ID NO: 1 or SEQ ID NO: 3 by deletion, substitution, insertion, and/or addition of one or more, and preferably one or several, such as 1 to 10, preferably 1 to 7, more preferably 1 to 5, further preferably 1 to 3, and still further preferably 1 or 2 amino acids and an amino acid sequence having at least 60%, at least 70%, at least 80%, at least 85%, preferably at least 90%, more preferably at least 95%, and particularly preferably at least 97%, 98%, or 99% sequence identity with the above amino acid sequence, when calculated with the use of known algorithms for homology search, such as BLAST or FASTA (based on, for example, default, (namely, initially set) parameters). Sequence identity among the enzymatic proteins comprising the amino acid sequences as shown in SEQ ID NO: 1 and SEQ ID NO: 3 is about 70% to 80%.

In this description, "sequence identity" is expressed in percentage (%) terms, representing the number of identical amino acids or nucleotides relative to the total number of amino acids or nucleotides containing gaps determined when, for example, two amino acid sequences or nucleotide sequences are aligned. (Gaps may or may not be introduced, and gaps are preferably introduced.)

The position-16-oxidizing enzyme according to the present invention encompasses a naturally occurring position-16-oxidizing enzyme isolated from a plant and a recombinant position-16-oxidizing enzyme produced via genetic engineering.

### 2. DNA encoding position-16-oxidizing enzyme

The term "DNA" used herein refers to any of genomic DNA, a gene, cDNA, and a chemically modified DNA.

DNA encoding a position-16-oxidizing enzyme that is used in the present invention is DNA encoding an enzyme having activity of oxidizing position 16 of the steroid skeleton.

Examples of DNAs encoding a position-16-oxidizing enzyme include DNA comprising the nucleotide sequence encoding the amino acid sequence as shown in SEQ ID NO: 1 and DNA comprising the nucleotide sequence encoding the amino acid sequence as shown in SEQ ID NO: 3. Specific examples thereof include DNA comprising the nucleotide sequence as shown in SEQ ID NO: 2 and DNA comprising the nucleotide sequence as shown in SEQ ID NO: 4. SEQ ID NO: 5 shows a sequence from the translation initiation codon to the termination codon of DNA of the St16DOX gene in the potato genome.

Examples of DNA encoding a position-16-oxidizing enzyme that can be used in the present invention include DNA hybridizing under stringent conditions to DNA comprising a nucleotide sequence complementary to the nucleotide sequence as shown in SEQ ID NO: 2, SEQ ID NO: 4, or SEQ ID NO: 5, DNA having at least 60%, at least 70%, at least 80%, at least 85%, preferably at least 90%, more preferably at least 95%, and particularly preferably at least 97%, 98%, or 99% sequence identity with the nucleotide sequence as shown in SEQ ID NO: 2, SEQ ID NO: 4, or SEQ ID NO: 5 calculated with the use of known algorithms for homology search, such as BLAST or FASTA (based on, for example, default (namely, initially set) parameters), and DNA encoding a protein comprising an amino acid sequence derived from the amino acid sequence of the protein encoded by any of the DNAs exemplified above by deletion, substitution, insertion, and/or addition of one or more, and preferably one or several amino acids, such as 1 to 10, preferably 1 to 7, more preferably 1 to 5, further preferably 1 to 3, and still further preferably 1 or 2 amino acids. DNA encoding a protein having activity of position-16-oxidizing enzyme is within the scope of such DNA.

Such DNA is a homolog, analog, or variant of DNA comprising the nucleotide sequence as shown in SEQ ID NO: 2, SEQ ID NO: 4, or SEQ ID NO: 5. Such DNA can be obtained from leaves, roots, seeds, or other parts of a plant that generates cholesterols, such as plants of the family *Solanaceae,* such as potatoes (*Solanum tuberosum*) and tomatoes (*Solanum lycopersicum*), via hybridization, PCR amplification, or other techniques.

Under "stringent conditions," DNAs having high degrees of sequence identity hybridize to each other, and a person skilled in the art can adequately determine such conditions. For example, stringent conditions comprise "1 x SSC, 0.1% SDS, 37°C," more stringent (moderate-stringency) conditions comprise "0.5 x SSC, 0.1% SDS, 42°C," and further stringent (high-stringency) conditions comprise "0.1 to 0.2 x SSC, 0.1% SDS, 65°C." Following hybridization, in addition, a step of washing may be carried out with, for example, 0.1 × SSC and 0.1% SDS at 55°C to 68°C, and the degree of stringency can then be increased. A 1 × SSC buffer is composed of 150 mM sodium chloride and 15 mM sodium citrate and has a pH of 7.0.

Hybridization conditions and PCR procedures are described in, for example, F. M. Ausbel et al., Short Protocols in Molecular Biology, 3rd ed., John Wiley & Sons, 1995.

A further example of DNA encoding a position-16-oxidizing enzyme that can be used in the present invention is DNA comprising a sequence resulting from the degeneracy of the genetic code of the nucleotide sequence as shown in SEQ ID NO: 2, SEQ ID NO: 4, or SEQ ID NO: 5 (i.e., a degenerate sequence).

As described above, DNA according to the present invention encodes a protein having activity of position-16-oxidizing enzyme; specifically, any of the proteins (a) to (c) below:
(a) a protein comprising the amino acid sequence as shown in SEQ ID NO: 1 or SEQ ID NO: 3;
(b) a protein comprising an amino acid sequence derived from the amino acid sequence as shown in SEQ ID NO: 1 or SEQ ID NO: 3 by deletion, substitution, insertion, or addition of one or several amino acids and having activity of position-16-oxidizing enzyme; and
(c) a protein comprising an amino acid sequence having 90% or higher sequence identity with the amino acid sequence as shown in SEQ ID NO: 1 or SEQ ID NO: 3 and having activity of position-16-oxidizing enzyme.

More specifically, DNA described above is selected from DNAs (d) to (g) below:
(d) DNA comprising the nucleotide sequence as shown in SEQ ID NO: 2 or SEQ ID NO: 4;
(e) DNA hybridizing under stringent conditions to DNA consisting of a nucleotide sequence complementary to DNA comprising the nucleotide sequence as shown in SEQ ID NO: 2 or SEQ ID NO: 4 and encoding a protein having activity of position-16-oxidizing enzyme;
(f) DNA comprising a nucleotide sequence having 90% or higher sequence identity with the nucleotide sequence as shown in SEQ ID NO: 2 or SEQ ID NO: 4 and encoding a protein having activity of position-16-oxidizing enzyme; and
(g) DNA comprising a degenerate sequence of the nucleotide sequence as shown in SEQ ID NO: 2 or SEQ ID NO: 4.

### 3. Recombinant vector

DNA according to the present invention is inserted expressibly into an adequate vector comprising a control sequence. The recombinant DNA thus obtained is a recombinant vector.

Any vectors that can be used in prokaryotic or eukaryotic cells can be used. Examples of vectors that can be used include microbial vectors of bacteria (e.g., *Escherichia, Pseudomonas, Bacillus*, and *Rhodococcus*), filamentous fungi (e.g., *Aspergillus, Neurospora, Fusarium, Trichoderma,* and *Penicillium*), *Basidiomycetes* (e.g., white-rot fungi), and yeast (e.g., *Saccharomyces, Pichia,* and *Candida*), plant cell vectors, and insect cell vectors.

Examples of bacterial vectors include pBR, pUC, pET, and pBluescript vectors. Examples of yeast vectors include, but are not limited to, pDR196, pYES-DEST 52, YIp5, YRp17, and YEp24 vectors. Examples of plant cell vectors include, but are not limited to, pGWB, pBiE12-GUS, pIG121-Hm, pBI121, pBiHyg-HSE, pB119, pBI101, pGV3850, and pABH-Hm1 vectors. Examples of insect cell vectors include, but are not limited to, pBM030, pBM034, and pBK283 vectors.

A constitutional element associated with gene expression, regulation, or secretion, such as a promoter, a terminator, an enhancer, the Shine-Dalgarno sequence, a ribosome-binding sequence, or a signal sequence, is incorporated into a vector used in the present invention, and a vector comprises a selection marker (e.g., a drug resistant gene or a reporter gene), according to need.

Examples of promoters include, but are not limited to, lac promoters, trp promoters, recA promoters, tac promoters, λPL promoters, T7 promoters, CaMV35S promoters, ADH1 promoters, GAL promoters, PHO5 promoters, PGK promoters, and GAPDH promoters.

Examples of drug resistant genes include kanamycin resistant genes, ampicillin resistant genes, and hygromycin resistant genes. Examples of reporter genes include lacZ genes, GFP genes, GUS genes, and luciferase genes. Examples of other selection markers include NPTII genes and dihydrofolate reductase genes.

It is preferable that a constitutional element associated with gene expression, regulation, or secretion be functionally incorporated into a recombinant vector in accordance with its properties. A person skilled in the art can adequately perform such procedure.

### 4. Method of gene suppression

The present invention provides a method for suppressing the expression of a gene encoding an enzyme that oxidizes position 16 of the steroid skeleton of a plant. Examples of methods for gene suppression that can be employed include the RNAi method via genetic recombination, the anti-sense method, the PTGS method involving the use of virus vectors, and direct introduction of small RNA. Also, methods for modifying the genomes, such as the zinc finger nuclease (ZFN) method, the tale nuclease (TALEN) method (Science, 333, 307, 2011), and Cre-loxP site-directed recombination, can be employed. Such methods occasionally involve the use of the sequence provided by the present invention as the site for direct introduction of a mutation. Alternatively, a sequence of a region adjacent to the sequence provided by the present invention may be identified based on sequence information, and the sequence of the adjacent region may be used, so as to delete the entire region of the gene encoding an enzyme that oxidizes position 16 of the steroid skeleton. In the present description, "suppression of gene expression" refers to reduction or elimination of RNA as a transcription product of a gene (DNA), a protein as a translation product, or enzymatic activity as a function of a protein. By suppressing the expression of a gene encoding an enzyme that oxidizes position 16 of the steroid skeleton, activity of the enzyme that oxidizes position 16 of the steroid skeleton of a plant is reduced or eliminated.

### 5. Selection of gene mutation, polymorphic mutant, and gene expression mutation

The present invention provides a method for detecting the presence of a mutation in a gene encoding an enzyme that oxidizes position 16 of the steroid skeleton, a polymorphism such as a single nucleotide polymorphism (SNP), and a gene expression mutation in a plant. A mutant may be obtained by radiation, chemical treatment, UV irradiation, or spontaneous mutation.

The above method comprises steps of: isolating genomic DNA and/or RNA from mutant plants, various plant varieties, and improved plant varieties and synthesizing cDNA from RNA by reverse transcription; amplifying a gene fragment containing a gene encoding an enzyme that oxidizes position 16 of the steroid skeleton from DNA using the DNA amplification technique; and determining the presence of a mutation in the DNA. A commercially available kit (such as DNeasy or RNeasy, Qiagen) can be used for a method of DNA or RNA extraction. A commercially available kit (such as a SuperScript First-Strand System, Invitrogen) can also be used for a method of cDNA synthesis. For a method of gene fragment amplification using a DNA amplification technique, so-called PCR, LAMP, and other techniques can be employed. These techniques involve the use of polymerase so as to amplify (i.e., to increase the number of copies of) a specific DNA sequence through continuous polymerase reactions. This reaction can be employed instead of cloning, and this requires only information concerning the nucleic acid sequence. In order to perform DNA amplification, primers complementary to the DNA sequence to be amplified are designed. Subsequently, the designed primers are produced by automatic DNA synthesis. DNA amplification techniques are well known in the art, and a person skilled in the art can readily implement such techniques based on the teachings and instructions provided in the present description. Some PCR techniques (and related techniques) are described in, for example, U.S. Patent Numbers 4,683,195, 4,683,202, 4,800,159, and 4,965,188 and "PCR Protocols: A guide to method and applications" edited by Innis et al.

In the step of determining the presence of a mutation or polymorphism in DNA, a detection method relying on homology between a mutant gene and a normal gene may be used. Examples of such method include the nucleotide sequencing (Applied Biosystems) and the TILLING method by which a mutant is detected using an enzyme that cleaves one member of a mismatched pair (Till et al., 2003, Genome Res 13: 524 to 530). The above method can be carried out by comparing the sequence data obtained by the above technique with the nucleotide sequence of a gene segment shown in SEQ ID NO: 2,4, or 5.

In the step of determining whether or not there is a difference in the amount of mRNA, cDNA may be subjected to quantitative PCR, such as RT-PCR or real-time PCR, using the primers produced based on the nucleotide sequence shown in SEQ ID NO: 2, 4, or 5. The results thereof may then be compared with the amount of cDNA obtained from the variety "Sassy," so that whether or not there is a difference in the amount of mRNA can be determined.

The genotypes and the phenotypes of the gene encoding an enzyme that oxidizes position 16 of the steroid skeleton of many wild-type potato plants or close relatives thereof remain unknown. By screening such wild-type plants, wild-type plants that have mutations in the biosynthetic genes and in which glycoalkaloid accumulation is undetectable or lower than that observed in cultivated plants or plants exhibiting lowered glycoalkaloid accumulation as a result of crossing can be selected.

Through the method of determining the presence of a mutation and/or a polymorphism, a mutation and/or a polymorphism in the gene encoding an enzyme that oxidizes position 16 of the steroid skeleton can be identified at the nucleotide level, and a plant comprising a mutation and/or a polymorphism in the gene encoding an enzyme that oxidizes position 16 of the steroid skeleton can be selected. The present invention encompasses a plant having a mutation and/or a polymorphism in the gene encoding an enzyme that oxidizes position 16 of the steroid skeleton that was obtained in the manner described above.

By determining that a mutation or polymorphism or a difference in the amount of mRNA exists, also, a plant in which the ability to express a gene encoding an enzyme that oxidizes position 16 of the steroid skeleton or the activity of an enzyme that oxidizes position 16 of the steroid skeleton is altered can be selected.

The ability to express a gene encoding an enzyme that oxidizes position 16 of the steroid skeleton or the activity of an enzyme that oxidizes position 16 of the steroid skeleton are suppressed by artificial mutagenesis, spontaneous mutagenesis, or genetic polymorphisms conserved in wild-type species. In this description, the expression "suppression of enzymatic activity" refers to reduction or elimination of the enzymatic activity. Suppression of activity of the enzyme that oxidizes position 16 of the steroid skeleton results in a situation in which normal functions of the enzyme that oxidizes position 16 of the steroid skeleton are lowered or lost.

Examples of such gene mutation include deletion of all or part of the gene encoding an enzyme that oxidizes position 16 of the steroid skeleton, substitution of some nucleotides with other nucleotides, and insertion of nucleotides. An example of insertion of nucleotides is insertion of several tens to several hundreds of continuous nucleotides into an exon of a gene encoding an enzyme that oxidizes position 16 of the steroid skeleton. Examples of substitution of some nucleotides with other nucleotides include substitution of a conserved sequence at the 5' splice site in an intron and insertion of a sequence that generates a new exon in an intron. Such substitution would inhibit the occurrence of normal splicing. Plants having such gene mutations are within the scope of the present invention. Further, the present invention comprises selecting a plant having such gene mutation as a mother plant. In addition, such plant is subjected to crossing as a mother plant, and a progeny thereof is selected. Thus, a cultivated plant with a lowered risk of glycoalkaloid accumulation can be prepared. The term "cultivated plant" used herein refers to a plant variety that can be subjected to cultivation aimed at crop production.

Also, a plant in which activity of an enzyme that oxidizes position 16 of the steroid skeleton is suppressed can be obtained by artificially modifying a gene encoding an enzyme that oxidizes position 16 of the steroid skeleton. The present invention also includes a method for producing a plant that can be obtained by artificially modifying a gene encoding an enzyme that oxidizes position 16 of the steroid skeleton and a plant produced by such method. Modification of the activity of an enzyme that oxidizes position 16 of the steroid skeleton of a particular plant via mutation is modification of an existing variety of such plant. While a wild-type plant is within the scope of such existing varieties, a naturally-occurring wild-type plant that is not used for industrial applications is not considered to be an existing variety. An existing variety refers to any of the plant varieties that are produced when the activity of an enzyme in such plant that oxidizes position 16 of the steroid skeleton is modified. That is, plant varieties produced by an artificial procedure, such as crossing or genetic engineering, are within the scope thereof. In the present invention, a plant is not required to exhibit enzymatic activity that is lower than that of every existing plant variety in order to fall under the category of "plant(s) in which the activity of an enzyme that oxidizes position 16 of the steroid skeleton is suppressed." As long as the activity of a plant is suppressed in comparison with that of a particular existing variety, such plant would fall under the category of "plant(s) in which the activity of an enzyme that oxidizes position 16 of the steroid skeleton is suppressed." A plant with the activity that is naturally suppressed via mutation without artificial procedure is within the scope of "plant(s) in which the activity of an enzyme that oxidizes position 16 of the steroid skeleton is suppressed." According to the method of the present invention, a plant in which the activity is naturally suppressed can be selected and established as a novel plant variety. When a particular existing plant variety is subjected to mutagenesis to prepare a plant in which activity of an enzyme that oxidizes position 16 of the steroid skeleton is suppressed, a control plant may be an existing variety that is the same as or different from the plant variety subjected to mutagenesis. Alternatively, a gene encoding an enzyme that oxidizes position 16 of the steroid skeleton selected from nature or produced via mutagenesis may be subjected to crossing with a gene of a plant having a mutation or polymorphism, so that the mutation of the gene encoding an enzyme that oxidizes position 16 of the steroid skeleton is fixed, and a novel plant variety with suppressed ability to express a gene encoding an enzyme that oxidizes position 16 of the steroid skeleton or activity of an enzyme that oxidizes position 16 of the steroid skeleton can be obtained.

When the plant is a potato *(Solanum tuberosum),* examples of existing varieties include "Cynthia," "Sassy," and "Cherie" (sold by JapanPotato), "Irish Cobbler (i.e., Danshaku)," "May Queen," and "Sayaka (Ministry of Agriculture, Forestry and Fisheries registration number: Norin No. 36)." A plant in which the ability to express a gene encoding an enzyme that oxidizes position 16 of the steroid skeleton or activity of an enzyme that oxidizes position 16 of the steroid skeleton is suppressed in comparison with an existing variety encompasses a plant in which the ability to express a gene encoding an enzyme that oxidizes position 16 of the steroid skeleton is lowered in comparison with an existing variety. Also, a plant in which the activity of an enzyme that oxidizes position 16 of the steroid skeleton is lowered in comparison with an existing variety is within the scope of the plant as described above. A plant in which the ability to express a gene encoding an enzyme that oxidizes position 16 of the steroid skeleton or activity of an enzyme that oxidizes position 16 of the steroid skeleton is suppressed in comparison with an existing variety is within the scope of the present invention.

In a plant in which the activity of an enzyme that oxidizes position 16 of the steroid skeleton is suppressed, the amount of an enzyme that oxidizes position 16 of the steroid skeleton synthesized is low, or such enzyme cannot be synthesized. Also, the content of the enzyme that oxidizes position 16 of the steroid skeleton is low in the plant, or such enzyme is absent in the plant. Alternatively, the activity of the enzyme that oxidizes position 16 of the steroid skeleton is low or eliminated. Accordingly, the plant has low glycoalkaloid content or lacks glycoalkaloids. In the case of a potato, for example, a glycoalkaloid such as chaconine or solanine is not synthesized, and thus the amount of a glycoalkaloid such as chaconine or solanine synthesized or existing in the potato tubers is low. In the case of a tomato, a glycoalkaloid such as tomatine is not synthesized, and thus the amount of a glycoalkaloid such as tomatine synthesized or existing in tomato fruit is low.

In a potato in which the activity of the enzyme that oxidizes position 16 of the steroid skeleton is low or lost, a glycoalkaloid such as chaconine or solanine is not synthesized in tubers, or the amount of a glycoalkaloid such as chaconine or solanine synthesized in tubers is lower than that in the existing variety described above. In such a case, also, the amount of a glycoalkaloid such as chaconine or solanine existing in tubers is low.

With the use of a plant having a mutation in a gene encoding an enzyme that oxidizes position 16 of the steroid skeleton (i.e., a mutant plant prepared by artificial modification of a gene encoding an enzyme that oxidizes position 16 of the steroid skeleton or a wild-type plant selected via screening) as a mother plant, a cultivated plant accumulating a reduced amount of or no glycoalkaloid and exhibiting good taste and cultivation properties can be produced. In the present invention, a plant in which "glycoalkaloid accumulation is reduced" is a cultivated plant in which the amount of glycoalkaloids accumulated or the rate of glycoalkaloid accumulation is lower than that of the mother plant used for the production thereof. In the present invention, a plant in which "glycoalkaloid accumulation is lost" is a cultivated plant in which the amount of glycoalkaloids becomes lower than the detection limit under conditions in which an existing plant variety would accumulate glycoalkaloids. In the present invention, glycoalkaloid accumulation is lowered or lost in the "cultivated plant in which a risk of glycoalkaloid accumulation is lowered."

### 6. Production of cultivated plant in which glycoalkaloid accumulation is lowered or lost via crossing

With the use of the variant obtained or the wild-type plant selected above as a mother plant, a cultivated plant in which glycoalkaloid accumulation is lowered or lost can be produced. If the mother plant is a variant obtained from a cultivated plant, it is considered to be effective to subject such variant to crossing or a variant having mutations at different sites of the same target gene to crossing, in order to fix the mutation of interest at an early stage. Concerning crossing with potatoes or close relatives thereof, damage such as classic self-incompatibility or incompatibility according to the Endosperm Balance Number (EBN) hypothesis has been known. Such plants may be subjected to processing, such as direct pollination of ovules, ovule culture, reciprocal crossing, or somatic cell fusion, so that crossing or processing equivalent thereto can be realized. Such processing may be implemented with reference to *"*Jagaimo Jiten (Dictionary of Potatoes)," 2012, edited by the Japan Root and Tuber Crops Development Association Inc. Foundation, Zenkoku Noson Kyoiku Kyokai, Co., Ltd. or "Handbook of Potato Production, Improvement, and Postharvest Management," 2006, edited by Gopal and Paul Khurana, pp. 77-108, Haworth Press Inc. When a wild-type plant is a parent plant having a gene with a mutation, the resulting plant is designated as a cultivated plant and subjected to backcrossing. Thus, a gene with a mutation can be introduced while maintaining good taste and excellent properties of cultivated plants. Also, the site of mutation in the gene may be analyzed at the nucleotide sequence level, so that a mutation-related gene marker can be obtained. With reference to the genomic information on, for example, the potato genome sequence (Nature, 2011; 475: 189-95) reported in 2011, in addition, a plurality of gene markers located in the vicinity of the gene of interest can be obtained, and progeny plants into which sites of mutation of interest have been selectively introduced can be efficiently selected. If particular markers are obtained in regions that cover the entire genome beyond regions in the vicinity of the gene of interest, a necessary portion (genetic region) can be selectively transferred. When there is genetic distance between a marker and a gene to be introduced (i.e., a trait), the marker may have a certain probability of differing from the trait. This requires a trait to be tested. However, genetic mutation discovered in the present invention is consistent with the relevant trait, and it is not necessary to test such trait. This enables conclusive testing of the seeds subjected crossing when the seeds germinate and DNAs are obtained. Testing involving the use of a DNA marker can be carried out with reference to, for example, *"*Genomu reberu no iden kaiseki (Genome-level genetic analysis) MAP and QTL," Yasuo Ukai, 2001, University of Tokyo Press. For example, the presence of the DNA markers can be determined with the use of polynucleotides, such as primers. A gene mutation test involving the use of a DNA marker can be carried out in combination with a trait test via glycoalkaloid analysis. When a cultivated plant is identified, in particular, it is preferable that reduction or elimination of glycoalkaloid accumulation be confirmed via analysis at each stage from plant growth to potato tuber storage.

### Examples

Hereafter, the present invention is described in greater detail with reference to the examples, although the present invention is not limited thereto.

### (Example 1) Extraction of gene of position-16-oxidizing enzyme from cDNA sequence of potato sprouts

Potato sprouts are known to accumulate a very large amount of glycoalkaloids. While a glycoalkaloid biosynthetic pathway remains unknown, cholesterol is considered to serve as a starting material. Thus, the potato sprouts were considered to be the excellent gene source to identify the gene of the position-16-oxidizing enzyme.

Following harvesting and storage for 3 months or longer, potatoes after a rest period were allowed to stand in the dark at 20°C and to sprout for about 2 weeks. The resulting sprouts were grounded with liquid nitrogen and total RNA was extracted with the use of RNeasy (Qiagen). cDNA was synthesized from 2 µg of RNA via reverse transcription with the use of a primer comprising a T7 promoter sequence added thereto. With the use of DNA polymerase and T7 RNA polymerase, the resulting fragment was amplified as RNA, and reverse transcription was carried out again to synthesize cDNA. With the use of an adaptor for GS FLX analysis, cDNA double-strands were synthesized therewith. After the synthesized double strands were physically cleaved to a fragment of several hundreds of base pairs, another adaptor for GS FLX analysis was ligated thereto to recover single-stranded DNA. With the use of the resultant as a template, approximately 150,000 expressed gene sequences were analyzed using the Genome Sequencer FLX System (assigned to Takara Bio, Inc.). As a result, the presence of as many as 174 expressed gene fragments (St16DOX) having the constitution of the 2-oxoglutaric-acid-dependent dioxygenase was discovered.

### (Example 2) Identification of St16DOX gene

With the use of the gene fragment mentioned above, primers: U997 (caccATGGCGGAGCTTCTTTCAAAC (SEQ ID NO: 6), "cacc" is an additional sequence used for cloning into a vector) and U998 (TTAAGCATCGATTTTGAAGGGC (SEQ ID NO: 7)) were synthesized in order to obtain a full-length sequence of St16DOX based on the sequence data SGN-U268477 in the Sol Genomics Network (http://solgenomics.net/index.pl). Total cDNA was synthesized from total RNA of Example 1 and applied to a SuperScript First-Strand System (Invitrogen). This cDNA was used as a template to amplify a gene by PCR at an annealing temperature of 55°C (30 cycles, with the use of PrimeSTAR HS DNA Polymerase, Takara Bio, Inc.). The resultant was cloned into the pENTR™/ D-TOPO Entry vector (Invitrogen). Polynucleotide sequences of the obtained 8 independent clones were determined. The determined sequence is shown in SEQ ID NO: 2, and the polypeptide sequence deduced based thereon is shown in SEQ ID NO: 1. The vector was designated as pTOPO-PSSt16DOX.

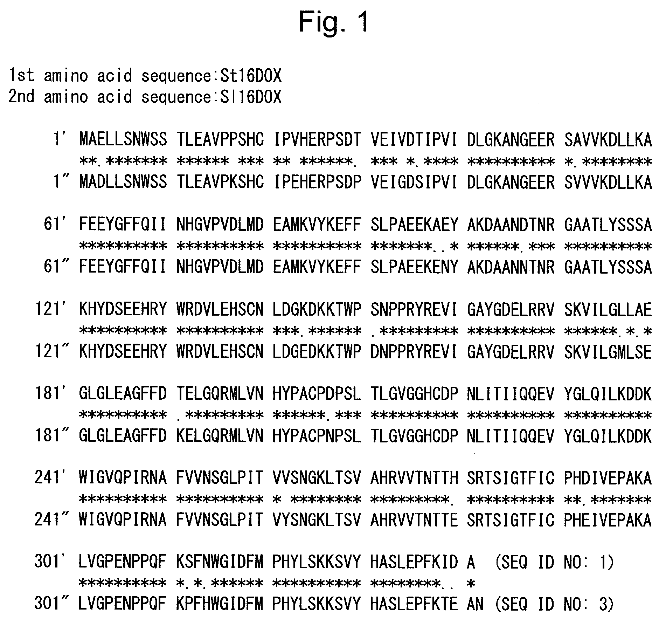

Further, the full-length gene sequence of the relevant potato was identified to be SGN-U581131 on the basis of the sequence information in the Sol Genomics Network (http://solgenomics.net/index.pl) was obtained. The identified sequence is as shown in SEQ ID NO: 4, and a polypeptide sequence deduced based thereon is as shown in SEQ ID NO: 3. Fig. 1 shows the alignments demonstrating the results of homology analysis between St16DOX and Sl16DOX, and amino acid sequence identity was very high (i.e., 93.3% for 341 amino acids).

### (Example 3) Identification of genomic gene of St16DOX gene

Genomic DNA was extracted from "Sassy" using DNeasy (Qiagen). PCR was carried out using the same primers as those used in Example 1 (U997: caccATGGCGGAGCTTCTTTCAAAC (SEQ ID NO: 6) and U998: TTAAGCATCGATTTTGAAGGGC (SEQ ID NO: 7)) to determine the nucleotide sequence of the full-length genomic DNA.

The genomic sequence of the potato gene was reported recently (Xu et al., Nature, 2011, 475: 189-197). The genomic sequence is disclosed on the website of the Potato Genome Sequencing Consortium Data Release (http://potatogenomics.plantbiology.msu.edu/index.html). On the basis of this sequence, the genomic gene of the St16DOX gene is found to be equivalent to PGSC0003DMG400011751. SEQ ID NO: 5 shows a sequence from the translation initiation codon to the termination codon of the St16DOX gene in the potato genome. As a result of comparison with SEQ ID NO: 2, the gene can be found to comprise 3 introns. While the genomic sequence of the tomato gene is disclosed on the website of the Sol Genomics Network (http://solgenomics.net/index.pl), SGN-U581131 is reported to be Solyc07g043420.2 on the chromosome 7. While the sequence is also reported to comprise 3 introns in an ORF, there is no description indicating functions.

### (Example 4) Vector construction for production of transformant in which St16DOX gene is suppressed

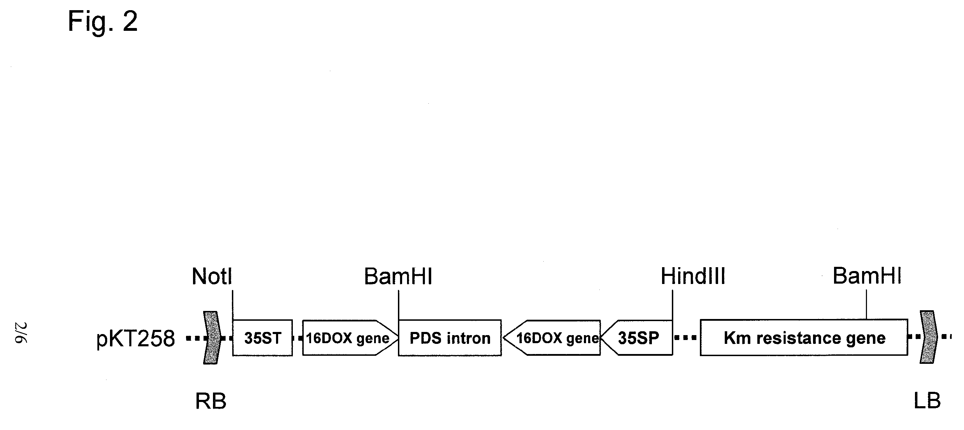

As to the method for suppressing the above gene by transformation, a reverse complementary gene fragment configured to be driven by a strong promoter was expressed (generally referred to as the RNAi method in plants) (Chuang and Meyerowitz, Proc. Natl. Acad. Sci" U.S.A., 97, 4985 to 90, 2000; Wesley et al., Plant J., 27, 581 to 90, 2001). The full-length cDNA obtained in Example 1 was subjected to PCR with the use of the primers (U1003: GAGCTCTAGATTTGGGAAAAGCTAATGGT (SEQ ID NO: 8) and U1004: GGATCCATATGCACCAATAACCTCC (SEQ ID NO: 9)) at an annealing temperature of 55°C (30 cycles, with the use of ExTaq DNA Polymerase, Takara Bio Inc.) to amplify the gene. The resultant was cloned into the pCR4-TOPO vector (Invitrogen) to obtain a gene fragment. Based on the binary vector pKT11 (JP 2001-161373 A), a vector pKT258 for plant transformation was prepared by ligating a cauliflower mosaic virus 35S RNA promoter, the above gene fragment (in the forward direction), the third intron of the *Arabidopsis thaliana* phytoene desaturase gene (AT4g14210), the above gene fragment (in the reverse direction), and a cauliflower mosaic virus 35S RNA terminator in this order (Fig. 2).

### (Example 5) Production of transformed potato plant

The vector prepared in Example 4 was introduced into the *Agrobacterium tumefaciens* GV3110 strain by the electroporation method (edited by Gelvin and Schilperoor, Plant Molecular Biology Manual, C2, 1 to 32, 1994, Kluwer Academic Publishers). The *Agrobacterium tumefaciens* GV3110 strain containing the vector was subjected to shaking culture at 28°C for 12 hours in a YEB liquid medium (5 g/l beef extract, 1 g/l yeast extract, 5 g/l peptone, 5 g/l sucrose, and 2 mM magnesium sulfate (pH 7.2)) containing 50 ppm kanamycin. The resulting culture solution (1.5 ml) was centrifuged at 10,000 rpm for 3 minutes to collect the bacteria, followed by washing in 1 ml of an LB medium for removal of kanamycin. Further, centrifugation was performed at 10,000 rpm for 3 minutes to collect the bacteria, the collected bacteria were resuspended in 1.5 ml of an MS medium containing 3% sucrose (Murashige and Skoog, Physiol. Plant., 15, 473 to 497, 1962), and the resultant was designated as a bacterial solution for infection.

Potato transformation was carried out according to the literature (Monma, 1990, Shokubutsu Soshiki Baiyo (Plant Tissue Culture) 7: 57 to 63). Microtubers obtained from the potato variety "Sassy" (Japan Agribio Co., Ltd.) were sliced to a thickness of 2 to 3 mm and provided as materials for *Agrobacterium* infection. The resulting slices were immersed in the aforementioned *Agrobacterial* solution and placed on sterilized filter paper so as to remove excess *Agrobacteria.* The slices were then placed on an MS medium (containing 1 ppm zeatin, 0.1 ppm IAA, 100 µM acetosyringone, and 0.8% agar) in a petri dish. The slices were then cultured under the conditions of 25°C, illumination for 16 hours (at a photon flux density of 32 µE/m²s)/non-illumination for 8 hours for 3 days. Subsequently, the slices were cultured in a medium containing 250 ppm carbenicillin in place of acetosyringone for 1 week. Thereafter, the slices were transferred onto a medium containing 50 ppm kanamycin, followed by subculture at 2-week intervals. During subculture, adventitious buds were formed and shoots grew. The elongated shoots were placed on an MS medium containing 250 ppm carbenicillin and 100 ppm kanamycin without plant growth regulators. A plant having a kanamycin-resistant gene as an exogenous gene was detected from among kanamycin-resistant, grown plants by subjecting the rooted shoots to PCR (conditions: 95°C for 5 minutes, 30 cycles of 95°C for 30 seconds, 55°C for 30 seconds, and 72°C for 1 minute, and 72°C for 10 minutes). Thus, the regenerated plant was confirmed to be a transformant. As the primers for specifically amplifying the kanamycin-resistant gene sequence, the following primers were used: TAAAGCACGAGGAAGCGGT (SEQ ID NO: 10) and GCACAACAGACAATCGGCT (SEQ ID NO: 11). Thus, 41 lines of transformed potato plants into which the vector pKT258 had been introduced were obtained.

### (Example 6) Analysis of glycoalkaloid content and 16DOX gene expression in plant transformant

The *in vitro* stems of the 41 plants obtained in Example 5 were elongated for 1 month after subculture. Thereafter, 2 to 4 of the elongated parts were put together to bring the amount thereof to about 100 mg, and the glycoalkaloid content therein was measured by the following method employing liquid chromatography with a column for alkali-resistant reversed-phase chromatography (JP 2011-27429 A). To the sample, 1 ml of 0.1% formic acid in 80% MeOH aq. was added, followed by pulverization using a mixer mill (1/25 sec, 10 min, 4°C). The pulverized products thus obtained were centrifuged at 10,000 rpm for 5 minutes at 4°C and then subjected to alcohol precipitation. The supernatant (25 µl) was fractionated, 475 µl of 0.1% aqueous formic acid was added, the mixture was filtered with the use of a multiscreen sorbinate (Millipore), and analysis was then carried out using LC-MS (LCMS-2010EV, Shimadzu Corporation; or Alliance e2795 Q-micro, Waters). LC conditions were analyzed via separation using a column XBridge^{™} Shield RP18-5 (diameter of 2.1 x 150 mm, Waters) and the mobile phase (a mobile phase A: 10 mM aqueous ammonium hydrogen carbonate (pH 10) and a mobile phase B: acetonitrile (40 : 60)) under the isocratic conditions (column oven: 40°C). Quantification was carried out using the standard products (chaconine and solanine, Sigma-Aldrich).

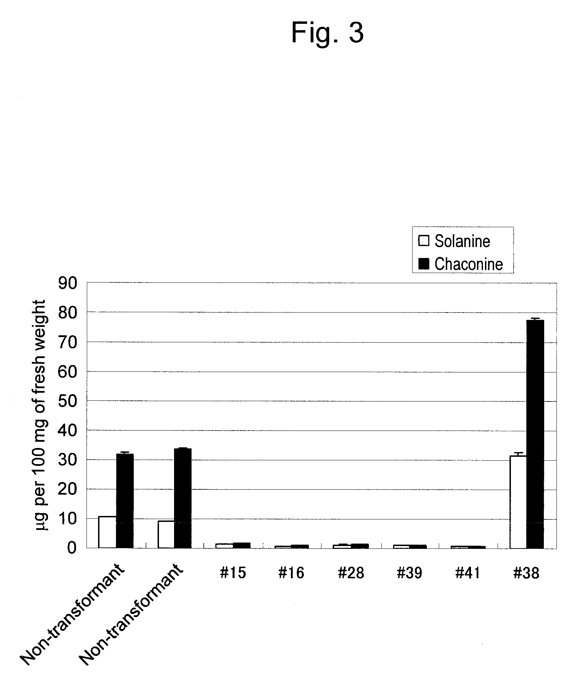

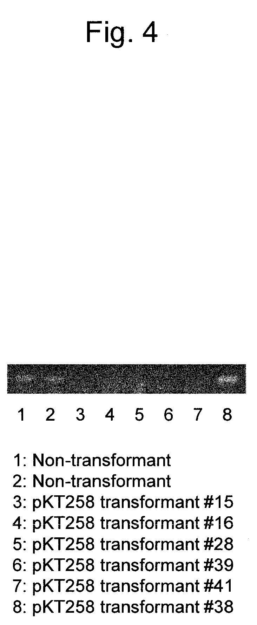

The *in vitro* stems of the 41 plants were analyzed twice. As a result, the amounts of glycoalkaloids accumulated in 5 lines (#5, #16, #28, #39, and #41) were found to be low with good reproducibility. The *in vitro* stems (about 200 mg) were collected from 5 lines in which the amount of glycoalkaloids accumulated was low, a line (#38) in which the amount of glycoalkaloids accumulated was not low, and 2 control plants into which the gene was not introduced. These *in vitro* stems were pulverized with liquid nitrogen, a half thereof was subjected to measurement of the glycoalkaloid content, and the other half was subjected to measurement of mRNA. The amount of glycoalkaloids accumulated in the 4 lines in which the amount of glycoalkaloids accumulated was low was considerably lower than that observed in the 2 non-transformed plants or the line in which the amount of glycoalkaloids accumulated was not low (Fig. 3). Total RNA was extracted from each plant using RNeasy (Qiagen), and total cDNA was synthesized using a SuperScript First-Strand System (Invitrogen). As a result of RT-PCR (PCR conditions: 95°C for 5 minutes and 20 cycles of 95°C for 30 seconds, 55°C for 30 seconds, and 72°C for 3 minutes) using the primers (U1060: TGGTTTACCAATTACGGTAGTTAGCA (SEQ ID NO: 12) and U998: TTAAGCATCGATTTTGAAGGGC (SEQ ID NO: 13)), it was found that mRNA expression of the 16DOX gene was extremely low or unobservable in 5 plants in which the amount of glycoalkaloids accumulated was low (Fig. 4). As a result, suppression of the 16DOX gene expression was found to result in an extremely reduced amount of glycoalkaloids accumulated, and the 16DOX gene was found to encode a glycoalkaloid biosynthetic enzyme that encodes an enzyme oxidizing position 16 of the steroid skeleton.

### (Example 7) Preparation of tuber from transformed plant of low-glycoalkaloid line

The *in vitro* plants of these 5 low-glycoalkaloid lines were amplified together with non-transformed plants, and 3 plants from each line were habituated to commercially available culture soil for vegetables, and the plants were then cultivated in a biohazard greenhouse according to a general method to obtain tubers. Each of these 5 lines (#5, #16, #28, #39, and #41) grew in a manner equivalent to the non-transformed plants, the total yield of #28 was somewhat lower, and equivalent tubers were obtained therefrom (Table 1).

**Table 1**

| Line No. | Number of tubers | S.D. | Average weight per tuber (g) | Total weight of plant (g) | S.D. |
|---|---|---|---|---|---|
| Non-transformant | 9.3 | 1.2 | 19.7 | 180.6 | 18.8 |
| #15 | 8.7 | 1.2 | 20.7 | 175.3 | 45.0 |
| #16 | 5.3 | 3.8 | 51.4 | 187.9 | 90.0 |
| #28 | 10.3 | 0.6 | 15.3 | 158.4 | 41.0 |
| #39 | 8.7 | 2.5 | 26.2 | 218.5 | 44.5 |
| #41 | 8.3 | 5.0 | 28.3 | 188.2 | 87.6 |

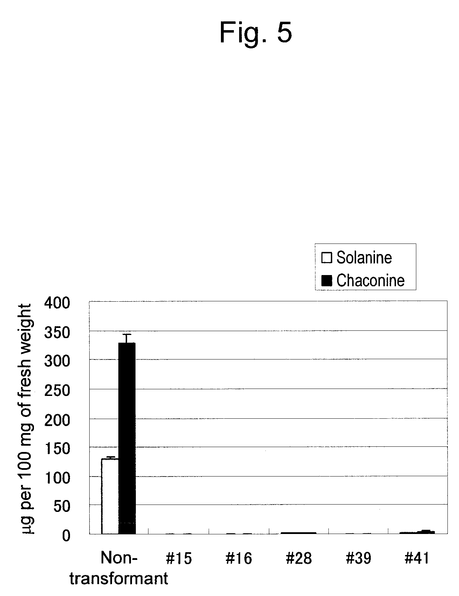

Further, the epidermis of the center part of each of the 3 harvested tubers was peeled with a thickness of about 1 mm, and the glycoalkaloid content was analyzed in the same manner. As a result, surprisingly, the glycoalkaloid content in the tubers was found to be extremely low (Fig. 5). These 4 transformed lines grew normally, and no traits such as dwarfism observed in *Arabidopsis thaliana* containing mutations in the DWF1 gene were observed. This indicates that there is a low degree of association between the 16DOX gene and the biosynthetic pathways of brassinolide, which is a plant hormone exhibiting a high degree of association with dwarfism, and that the 16DOX gene is a gene encoding an enzyme that oxidizes position 16 of the steroid skeleton exhibiting a high degree of association with the biosynthetic pathway of glycoalkaloids.

### (Example 8) Production of transformed tomato plant

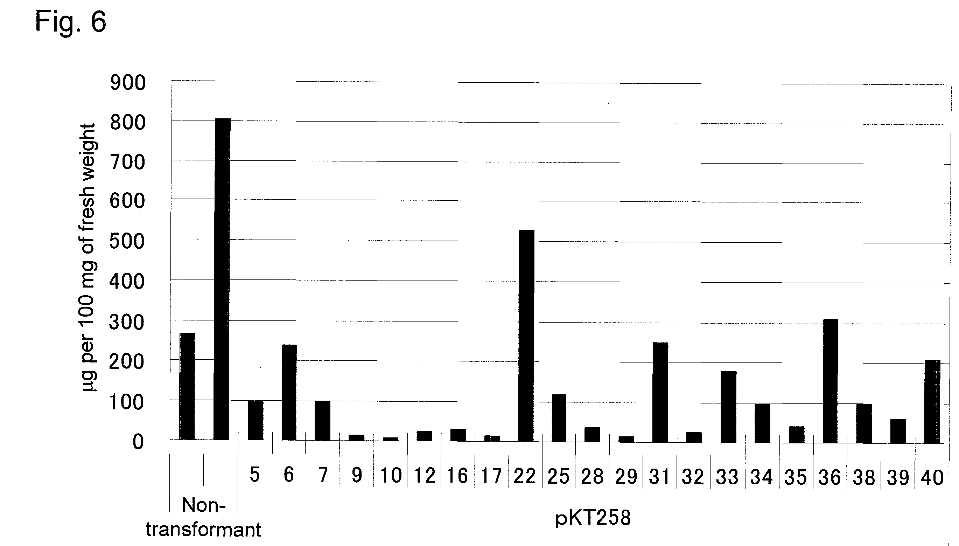

Tomato transformation was performed according to the literature (Sun et al., 2006, Plant Cell Physiol., 47: 426-431). The *Agrobacterium tumefaciens* GV3110 strain containing the vector pKT258 prepared in (Example 4) was cultured to give a bacterial solution for infection. Sections of 5 mm or smaller taken from the cotyledons of the tomato line (*Solanum lycopersicum*) called "Ailsa Craig" (provided by the National BioResource Project-Tomato (NBRP)) obtained by sterile seeding were immersed in the aforementioned *Agrobacterial* suspension for 10 minutes to allow infection to take place. The leaves were then placed on sterilized filter paper to remove excess *Agrobacteria.* The leaves were placed on a coculture MS medium (containing 1.5 mg/l zeatin, 40 µM acetosyringone, and 0.3% Gelrite^{®}) (Murashige and Skoog, Physiol. Plant., 15, 473 to 497, 1962) in a petri dish. The petri dish was placed in a dark place and culture was performed at 25°C for 3 days. The sections were subjected to subculture at 2-week intervals in a selective MS medium 1 (containing 1.5 mg/l zeatin, 100 mg/l kanamycin, 375 mg/l augmentin, and 0.3% Gelrite^{®}) under the conditions of illumination for 16 hours (at a photon flux density: 32 µE/m²s)/non-illumination for 8 hours at 25°C. During subculture, adventitious buds were formed and shoots grew. In order to further elongate the shoots, the shoots were transplanted to a selective MS medium 2 (containing 1.0 mg/l zeatin, 100 mg/l kanamycin, 375 mg/l augmentin, and 0.3% Gelrite^{®}). The elongated shoots were allowed to root in a selective 1/2 concentration MS medium (containing 100 mg/l kanamycin, 375 mg/l augmentin, and 0.3% Gelrite^{®}). A plant having a kanamycin-resistant gene as an exogenous gene was detected from among kanamycin-resistant, grown plants by subjecting the shoots to PCR using primers that specifically amplify the sequence of the kanamycin-resistant gene. Thus, the regenerated plant was confirmed to be a transformant. The transformed tomato plants (21 lines) into which the vector pKT258 had been introduced were obtained. From each of newly developed young leaves of the resulting 21 *in vitro* lines, about 100 mg was weighed out, and the glycoalkaloid content (i.e., the α tomatine content; the standard α tomatine product of Sigma Aldrich was used) was measured by the method employed in Example 6 in a manner similar to that for potatoes, except that analysis was performed with a ratio of mobile phase A to mobile phase B of 60:40. Among the 21 plants, tomatine content was remarkably low in 9 plants, which was 1/10 or less (i.e., 53 µg or less) of 536 µg per 100 mg fresh weight, which was the average of control plants (Fig. 6). These transformed lines grew normally.

### (Example 9) Production of potato containing mutations in St16DOX gene

Self-propagating seeds of the potato variety "*Hokkaikogane*" were subjected to mutation treatment by means of particle beam irradiation (an NIRS-HIMAC irradiation apparatus; Radiation Research 154, 485-496, 2000), a 90 to 470 Gy neon ion beam (30 kev/µm), and a 20 to 80 Gy iron ion beam (185 kev/µm). The seeds were immersed in a solution containing 2,000 ppm of gibberellin (Kyowa Hakko Kirin Co., Ltd.) for 2 days, and the resultants were sowed in commercially available culture soil. Culture was conducted for a photoperiod of 14 hours at a temperature of 23°C, and the lengths thereof reached approximately 3 cm and 4 foliage leaves developed approximately 20 days later. The shoot apex was then cut back to a point just above the lowest foliage leaf. Approximately 2 weeks thereafter, a lateral bud extended from the cut-back node, and DNA was extracted from the leaf developed from the lateral bud. Leaves of the 4 plants were collectively used as a single sample, 450 µl of an extraction liquid (0.1 M Tris-HCl, pH 8.5, 40 mM EDTA, 0.6 M NaCl) was added thereto, and the resultant was pulverized using zirconia beads and a mixer mill (Retsch) (oscillation cycle 1/25 sec, 5 min). A Proteinase K solution (10 mg/ml) was added in an amount of 10 µl, and treatment was then carried out at 55°C for 20 minutes. Thereafter, 160 µl of 5 M potassium acetate was added and centrifugation was carried out at 15,000 rpm for 5 minutes. The supernatant (100 µl) was precipitated in the equivalent amount of isopropanol, and the resultant was dissolved in 100 µl of a TE solution (10 mM Tris-HCl, pH 8.0, 1 mM EDTA) to prepare pooled DNA samples. Mutation was detected by the Tilling method (Nature Protocol 1, 2465-2477).

By designing primers specific for each allele of the St16DOX gene of *Hokkaikogane* on the basis of SEQ ID NO: 2 and SEQ ID NO: 5, an exon of each 16DOX gene can be amplified. DNA is amplified by PCR (conditions: 95°C for 5 minutes, 30 cycles of 95°C for 30 seconds, 55°C for 30 seconds, and 72°C for 1 minute, and 72°C for 5 minutes), denatured at 98°C for 2 minutes, and then reassociated. Thereafter, a solution containing CEL1 nuclease (Nature Protocol 1, 2465-2477) is added, and treatment is carried out at 45°C for 15 minutes. The DNA product is subjected to electrophoresis on 8% acrylamide gel or electrophoresis with the use of MultiNA (Shimadzu Corporation) and the DNA-1000 Kit (Shimadzu Corporation), and plants comprising mutation can be detected as bands that had newly emerged. From the pooled DNA samples in which mutation had been detected, DNA is individually extracted again, and mutants can be identified by the Tilling method. Concerning a plant that had undergone point mutation, adequate primers may be designed based on the sequence as shown in SEQ ID NO: 2, and the site of mutation can be identified. As a result, gene-disruption mutants can be selected.

### Industrial Applicability

The present invention can provide a plant belonging to the family *Solanaceae,* such as a potato, that does not accumulate glycoalkaloids therein.

### Sequence Listing Free Text

SEQ ID NOs: 6 to 13: Primers

All publications, patents, and patent applications cited herein are incorporated herein by reference in their entirety.

## Claims

1. A method for producing a plant with a reduced risk for accumulation of glycoalkaloids, comprising suppressing the activity of an enzyme that oxidizes position 16 of the steroid skeleton of a plant belonging to the family *Solanaceae* or suppressing the expression of a gene encoding the enzyme.

2. The method according to claim 1, wherein the plant is a cultivated plant.

3. The method according to claim 1 or 2, wherein the enzyme that oxidizes position 16 of the steroid skeleton of a plant belonging to the family *Solanaceae* is either protein (a) or (b) below:
(a) a protein consisting of the amino acid sequence as shown in SEQ ID NO: 1 or 3; or
(b) a protein consisting of an amino acid sequence derived from the amino acid sequence as shown in SEQ ID NO: 1 or 3 by deletion, substitution, insertion, or addition of one or several amino acids and oxidizing position 16 of the steroid skeleton of a plant belonging to the family *Solanaceae.*

4. The method according to claim 1 or 2, wherein the enzyme that oxidizes position 16 of the steroid skeleton of a plant belonging to the family *Solanaceae* is encoded by a gene consisting of any of DNAs (c) to (f) below:
(c) DNA consisting of the nucleotide sequence as shown in SEQ ID NO: 2 or 4;
(d) DNA hybridizing under stringent conditions to DNA consisting of a nucleotide sequence complementary to DNA consisting of the nucleotide sequence as shown in SEQ ID NO: 2 or 4 and encoding a protein having activity of oxidizing position 16 of the steroid skeleton of a plant belonging to the family *Solanaceae*;
(e) DNA consisting of a nucleotide sequence having 80% or higher sequence identity with the nucleotide sequence as shown in SEQ ID NO: 2 or 4 and encoding a protein that oxidizes position 16 of the steroid skeleton of a plant belonging to the family *Solanaceae;* and
(f) DNA consisting of a degenerate isomer comprising the nucleotide sequence as shown in SEQ ID NO: 2 or 4.

5. The method according to claim 1 or 2, wherein the enzyme that oxidizes position 16 of the steroid skeleton of a plant belonging to the family *Solanaceae* is protein (g) or (h) below:
(g) a protein consisting of the amino acid sequence as shown in SEQ ID NO: 3; or
(h) a protein consisting of an amino acid sequence derived from the amino acid sequence as shown in SEQ ID NO: 3 by deletion, substitution, insertion, or addition of one or several amino acids and oxidizing position 16 of the steroid skeleton of a plant belonging to the family *Solanaceae.*

6. The method according to claim 1 or 2, wherein the enzyme that oxidizes position 16 of the steroid skeleton of a plant belonging to the family *Solanaceae* is encoded by a gene comprising any of DNAs (i) to (1) below:
(i) DNA consisting of the nucleotide sequence as shown in SEQ ID NO: 4;
(j) DNA hybridizing under stringent conditions to DNA consisting of a nucleotide sequence complementary to DNA consisting of the nucleotide sequence as shown in SEQ ID NO: 4 and encoding a protein that oxidizes position 16 of the steroid skeleton of a plant belonging to the family *Solanaceae*;
(k) DNA consisting of a nucleotide sequence having 80% or higher homology to the nucleotide sequence as shown in SEQ ID NO: 4 and encoding a protein that oxidizes position 16 of the steroid skeleton of a plant belonging to the family *Solanaceae*; and
(1) DNA consisting of a degenerate isomer of the nucleotide sequence as shown in SEQ ID NO: 4.

7. The method according to any one of claims 1 to 6, wherein the activity of the enzyme that oxidizes position 16 of the steroid skeleton of a plant belonging to the family *Solanaceae* or expression of a gene encoding such enzyme is suppressed by genetic recombination.

8. The method according to any one of claims 1 to 6, wherein the activity of the enzyme that oxidizes position 16 of the steroid skeleton of a plant belonging to the family *Solanaceae* or expression of a gene encoding such enzyme is suppressed by deletion of a gene that encodes the enzyme.

9. The method according to any one of claims 1 to 6, which comprises selecting a progeny obtained by crossing involving the use of a plant in which the activity of the enzyme that oxidizes position 16 of the steroid skeleton of a plant belonging to the family *Solanaceae* or expression of a gene encoding such enzyme is suppressed as a mother plant.

10. The method according to claim 9, wherein the mother plant is obtained by artificial modification of the gene encoding an enzyme that oxidizes position 16 of the steroid skeleton of a plant belonging to the family *Solanaceae* via mutation.

11. The method according to claim 9, wherein the mother plant is obtained by screening of wild-type plants.

12. The method according to claim 9, wherein the mother plant is obtained and the progeny is selected by detecting the presence of a mutation or polymorphism in the gene encoding an enzyme that oxidizes position 16 of the steroid skeleton of a plant belonging to the family *Solanaceae.*

13. The method according to claim 12, wherein the detection of the presence of a mutation or polymorphism comprises steps of:
(i) isolating a nucleic acid, which is genomic DNA or RNA, from a plant;
(ii) synthesizing cDNA by reverse transcription when the nucleic acid of (i) is RNA;
(iii) amplifying a gene fragment comprising at least a part of the nucleotide sequence as shown in SEQ ID NO: 2, 4, or 5 from DNA obtained in the step (i) or (ii); and
(iv) determining the presence of a mutation or polymorphism in DNA.

14. A cultivated plant produced by the method according to any one of claims 1 to 13.

15. The cultivated plant according to claim 14, which is a potato.

16. A gene marker sequence used in the method according to claim 13.

17. A primer sequence for gene amplification used in the method according to claim 13.

18. A cultivated plant comprising a mutation in a gene encoding an enzyme that oxidizes position 16 of the steroid skeleton of a plant belonging to the family *Solanaceae* and having a reduced risk of accumulation of glycoalkaloids.

19. The cultivated plant according to claim 18, which does not accumulate glycoalkaloids.
